# DEMANDE DE BREVET EUROPEEN

(11) **EP 3 748 642 A1**
(43) Date de publication de la demande: **09.12.2020**
(21) Numéro de dépôt: 19177948.7
(22) Date de dépôt: 03.06.2019
(51) Int. Cl.: G16H 20/70

(54) **DISPOSITIF D'AIDE A LA DECISION MEDICALE**

(71) Demandeur: Université de Rennes 1, 35000 Rennes (FR); Centre Eugène Marquis, 35042 Rennes (FR); INSERM (Institut National de la Santé et de la Recherche Médicale), 75013 Paris (FR)
(72) Inventeur: BEZY-WENDLING, Johanne, 35235 Thorigne Fouillard (FR); SAINT-JALMES, Hervé, 35200 Rennes (FR); ROLLAND, Yan, 35200 Rennes (FR); ELIAT, Pierre-Antoine, 35650 Le Rheu (FR); CUTRI, Elena, 35000 Rennes (FR)
(74) Mandataire: Plasseraud IP

(57) **Abrégé**

Dispositif médical comprenant une unité de commande configurée pour déterminer et afficher dynamiquement sur un dispositif d'affichage une information relative à une injection d'un agent actif dans un cathéter positionné ou positionnable dans un patient, l'information étant déterminée en fonction de :
- au moins une modélisation géométrique d'un arbre vasculaire du patient, alimentant une ou plusieurs tumeurs,
- au moins un paramètre interventionnel susceptible d'évoluer de façon dynamique et choisi parmi un type de cathéter, une position de cathéter, une vitesse d'injection, un type d'agent actif, une quantité d'agent actif et le nombre d'injections.

## Description

### Domaine technique

L'invention relève du domaine de l'aide à la décision médicale. Elle trouve une application dans le domaine du traitement du carcinome hépatocellulaire (CHC).

Les options de traitement du CHC sont limitées. Seulement 15% des patients peuvent être traités de façon chirurgicale, 50 % par des traitements non-chirurgicaux (ablation percutanée par radiofréquences, chimiothérapie ou radiothérapie) et 35% par des traitements palliatifs.

Une alternative particulièrement intéressante pour éviter les effets secondaires de la chimiothérapie et la radiothérapie est la radiothérapie métabolique interne (RTMI), qui consiste en l'injection de millions de microsphères marquées par exemple à l'Yttrium 90 dans l'artère hépatique, en amont de la tumeur. L'administration de cette thérapie exige une connaissance précise de données spécifiques (géométries et flux) de l'arbre artériel hépatique de chaque patient.

Toutefois, un résultat sous-optimal de la procédure se produit souvent : un ciblage non efficace de la tumeur est obtenu qui, à son tour, provoque une irradiation dans le tissu sain environnant. Ce dernier peut provoquer des effets secondaires chez les patients. Différents facteurs peuvent jouer un rôle crucial dans l'optimisation du traitement du RTMI, comme la position exacte du cathéter, la forme du cathéter, la vitesse d'injection des sphères et la géométrie particulière des vaisseaux. Ces facteurs ne sont pas contrôlables a priori avec les techniques de l'art antérieur.

Le succès de l'intervention, à ce jour, est fortement lié à l'expérience clinique et à l'expertise du médecin.

Actuellement, il n'existe aucun outil permettant d'aider les médecins à identifier la position et la configuration optimale du cathéter pour cibler la tumeur lors de l'injection des microsphères. Le site d'injection est actuellement validé lors d'une première intervention à but diagnostique par l'injection des macros-agrégats d'albumine (MAA) chargés au Technétium 99m, suivie d'une scintigraphie SPECT/CT permettant de déterminer et quantifier la fixation du matériel radioactif. Après cette première intervention, et en l'absence de contre-indication, le médecin injecte les sphères au cours d'une artériographie thérapeutique en salle de radiologie interventionnelle une ou deux semaines plus tard, et une deuxième scintigraphie est réalisée une heure après pour connaitre l'étendue du foie effectivement traitée. Des images CT ou IRM sont réalisées dans les mois suivants dans le but de contrôler l'évolution de la tumeur.

Malgré l'évaluation effectuée lors du prétraitement, il peut exister une discordance dans la distribution des protéines (MAA) et des microsphères, ce qui entraîne au mieux une efficacité sous-optimale du RTMI.

Différents facteurs peuvent jouer un rôle crucial dans l'efficacité du traitement par RTMI, comme la position exacte du cathéter, la forme du cathéter, la vitesse d'injection, et la géométrie particulière des vaisseaux ou des changements dans l'arbre hépatique par exemple en raison d'un spasme. Ces facteurs ne sont actuellement pas contrôlables a priori.

Il existe donc un besoin pour un outil d'aide à la décision dans le cas des traitements intraartériels tels que la RTMI, en particulier dans le cas du traitement du CHC.

A cet effet, un premier objet de l'invention concerne un Dispositif médical comprenant une unité de commande configurée pour déterminer et afficher dynamiquement sur un dispositif d'affichage une information relative à une injection d'un agent actif dans un cathéter positionné ou positionnable dans un patient, l'information étant déterminée en fonction de :
- au moins une modélisation géométrique d'un arbre vasculaire du patient, de préférence un arbre hépatique, alimentant une ou plusieurs tumeurs,
- au moins un paramètre interventionnel susceptible d'évoluer de façon dynamique et choisi parmi un type de cathéter, une position de cathéter, une vitesse d'injection, un type d'agent actif, une quantité d'agent actif et le nombre d'injections.

L'invention adresse le problème d'aider le clinicien en temps réel lors de son processus -de décision visant à mieux cibler la tumeur tout en limitant la délivrance de l'agent actif au tissu sain, optimisant de ce fait le traitement. L'invention a pour but également de permettre la formation du personnel médical et plus généralement du personnel impliqué dans le traitement RTMI à travers l'utilisation de l'outil selon l'invention. Enfin, l'invention peut également permettre aux fabricants de cathéters et de sphères radioactives de tester différentes configurations, et de développer de nouvelles approches en faisant varier la taille des billes, en mélangeant plusieurs tailles de billes, en faisant varier la forme et la rigidité des cathéters etc.

L'invention offre au médecin la possibilité de procéder en temps réel à l'évaluation de la procédure optimale. Le clinicien peut évaluer l'effet des différents cathéters utilisés dans la routine clinique, afin d'évaluer le site d'injection optimal, le tout de manière simple et intuitive. Le clinicien peut rejouer la simulation avec d'autres conditions (position, vitesse, angle d'inclinaison, ...) s'il n'est pas satisfait du résultat, jusqu'à obtenir un résultat satisfaisant. Le clinicien peut enfin effectuer le geste optimal trouvé.

L'invention peut également présenter une ou plusieurs des caractéristiques ci-dessous prises indépendamment ou en combinaison.

L'étape de modélisation géométrique peut comporter une première sous étape d'obtention de l'image tridimensionnelle par des méthodes de métrologie et de l'instrumentation dédiée dans les domaines de l'imagerie et de la spectroscopie par résonance magnétique (IRM / SRM).

L'étape de modélisation géométrique peut comporter une deuxième sous étape comprenant la segmentation de l'arbre vasculaire, par exemple en déterminant un modèle anatomique tridimensionnel virtuel du patient comprenant la structure vasculaire de l'organe (le foie dans le cas de CHC). Ce modèle est par exemple obtenu par application d'algorithmes automatiques ou semi-automatiques.

Il est possible d'estimer l'emplacement et le volume de la tumeur en utilisant des images du scanner CT ou par IRM ou par Imagerie Nucléaire ou par ultrasons.

L'information est de préférence également déterminée en fonction de données mesurées sur le patient et de préférence choisies parmi une pression artérielle, une vélocité du sang et une distribution d'un agent actif.

L'obtention des données spécifiques au patient peut comporter l'utilisation d'IRM de flux et de capteurs de pression.

L'unité de commande est de préférence configurée pour mettre en oeuvre un modèle de mécanique des fluides et de transport de masse.

Par exemple, la modélisation géométrique d'un arbre vasculaire du patient, de préférence un arbre hépatique, alimentant une ou plusieurs tumeurs peut comporter une étape de mise en place d'un modèle en temps réel et paramétré visant à déterminer dynamiquement l'information. Cette étape facultative de mise en place d'un modèle en temps réel et paramétré comporte premièrement la construction d'un modèle de mécanique des fluides computationnels décrivant soit le flux soit le transport de masses, en particulier des particules injectées. Il est alors possible de simuler l'injection des particules par un cathéter situé dans l'arbre vasculaire.

L'information peut être déterminée en prenant en compte un résultat d'au moins une simulation réalisée par une méthode d'analyse de préférence par des volumes finis ou par des éléments finis ou par des modèles Lattice-Boltzmann. Ce résultat peut par exemple permettre de mettre en place le modèle en temps réel et paramétré.

Cette étape peut comporter aussi la définition des conditions aux limites basées sur des données spécifiques au patient et la définition de façon paramétrique des paramètres d'injection.

La définition du ou des paramètres interventionnels susceptibles d'évoluer de façon dynamique peut passer par le choix et la définition de paramètres géométriques pouvant être modifiés de façon automatique et la création de la discrétisation spatiale (c'est-à-dire le maillage) pour des simulations numériques. La création du maillage implique son optimisation pour réduire les temps et les coûts de calcul.

La détermination de l'information relative à une injection d'un agent actif dans un cathéter positionné ou positionnable dans un patient peut comporter l'identification des paramètres cibles qui sont des indicateurs d'un traitement réussi.

L'information peut être déterminée en prenant en compte par exemple un modèle d'ordre réduit. Par exemple, l'évaluation des paramètres d'injection optimaux en temps réel peut impliquer l'utilisation des modèles d'ordre réduit pour exécuter automatiquement l'analyse d'optimisation dans un espace de conception raisonnable c'est-à-dire l'espace défini par la combinaison des différents paramètres, sans qu'il soit nécessaire de réaliser une simulation pour chaque combinaison des paramètres. L'utilisation de modèles d'ordre réduit permet d'effectuer plusieurs calculs en mobilisant suffisamment peu de ressources pour exploiter les résultats du modèle en faisant varier les paramètres d'intérêt, par exemple, la dimension et les positions du cathéter. Le temps de calcul s'en trouve donc avantageusement réduit.

L'information peut également être déterminée en fonction d'images du patient, les images étant de préférence obtenues par CT ou par IRM ou par Imagerie Nucléaire ou par ultrasons.

L'affichage dynamique de l'information en temps réel prévoit de préférence soit l'utilisation de calculs off-line effectués par les modèles d'ordre réduit soit l'utilisation de méthodes de type Lattice-Boltzmann. De cette façon, les configurations d'injection optimales peuvent être affichées.

Différents types d'agents actifs peuvent être utilisés dans le cadre de l'invention. Il peut s'agir d'agents radiomarqués tels que des macros-agrégats d'albumine (MAA) marqués au Tc-99m. Il peut également s'agir de molécules de chimiothérapie mélangées à du lipiodol ® dans le cadre d'une chimioembolisation. Il peut encore s'agir d'agents d'embolisation, par exemple les particules commercialisées sous la référence EmboCept® S, Embosphere® Microspheres, ou encore des particules d'embolisation à élution de médicament telles que celles commercialisées sous les références HepaSphere™ Microspheres - Merit Médical EMEA, DC Bead®. Enfin, il peut aussi s'agir d'agents de contraste.

L'agent actif, de préférence radioactif, est de préférence choisi parmi des particules comportant de l'Yttrium 90, des particules comportant de l'Holmium 166, et un fluide comportant du Rhenium 188.

Ces particules sont particulièrement efficaces dans le cas de traitements intraartériels, notamment de type RTMI.

Les particules comportant de l'Yttrium 90 sont par exemples les particules commercialisées sous la référence SIR-Speres® Y-90 ou sous la référence TheraSphere.

Les particules comportant de l'Holmium 166 sont par exemples les particules commercialisées sous la référence QuiremSpheres® microspheres.

Les fluides comportant du Rhenium 188 sont par exemples ceux commercialisés sous la référence 188Re-HDD-Lipiodol.

Le cathéter est par exemple choisi parmi un cathéter de type smart micro-cathéter, et un cathéter anti-reflux.

L'invention peut permettre de choisir parmi différents cathéters. Des cathéters utilisables dans le cadre de l'invention sont par exemple ceux commercialisés sous la référence MeritMaestro® microcatheter (diameter 2.4F), sous la référence Terumo Progeat® microcatheter (diameter 2.4F), ou encore sous la référence -SwiftNinja®.

Le dispositif selon l'invention peut être configuré pour déterminer l'information lorsque le cathéter n'est pas positionné dans le patient. Dans ce cas, l'information à déterminer est de préférence une position du cathéter. L'invention permet alors d'aider au choix du positionnement du cathéter afin d'optimiser le ciblage.

Le dispositif selon l'invention peut aussi être configuré pour déterminer l'information lorsque le cathéter est positionné dans le patient. Dans ce cas, l'information à déterminer est de préférence une quantité d'agent actif libéré. Cela permet avantageusement de déterminer une quantité d'agent actif à libérer adaptée à l'arbre vasculaire qui sera irrigué, notamment en fonction de la position du cathéter.

Le patient est par exemple porteur d'une ou plusieurs tumeurs, de préférence un carcinome hépatocellulaire. En effet, l'invention est particulièrement utile lorsque l'agent actif est susceptible d'être libéré dans un arbre vasculaire complexe et variable d'un individu à l'autre, comme l'arbre hépatique.

De préférence, l'information déterminée est choisie parmi des combinaisons des paramètres d'injection qui permettent le ciblage le plus efficace de la tumeur.

Un autre objet de l'invention concerne un programme informatique comportant des instructions pour la détermination et l'affichage dynamique sur un dispositif d'affichage d'une information relative à une injection d'un agent actif dans un cathéter positionné ou positionnable dans un patient, l'information étant déterminée en fonction de :
- au moins une modélisation géométrique d'un arbre hépatique du patient,
- au moins un paramètre chirurgical susceptible d'évoluer de façon dynamique et choisi parmi un type de cathéter, une position de cathéter, une vitesse d'injection, un type d'agent actif et une quantité d'agent actif ;
lorsque ce programme est exécuté par au moins un processeur.

Un autre objet de l'invention concerne un support d'enregistrement non transitoire lisible par un ordinateur sur lequel est enregistré un programme selon l'invention.

L'invention a également pour objet un procédé pré-opératoire d'aide à la décision médicale comportant une étape de détermination d'une information relative à une injection d'un agent actif dans un cathéter positionnable dans un patient, l'information étant déterminée en fonction de :
- au moins une modélisation géométrique d'un arbre hépatique du patient,
- au moins un paramètre chirurgical susceptible d'évoluer de façon dynamique et choisi parmi un type de cathéter, une position de cathéter, une vitesse d'injection, un type d'agent actif et une quantité d'agent actif.

### Brève description des dessins

D'autres caractéristiques, détails et avantages de l'invention apparaîtront à la lecture de la description détaillée ci-après, et à l'analyse des dessins annexés, sur lesquels :
**Fig. 1**

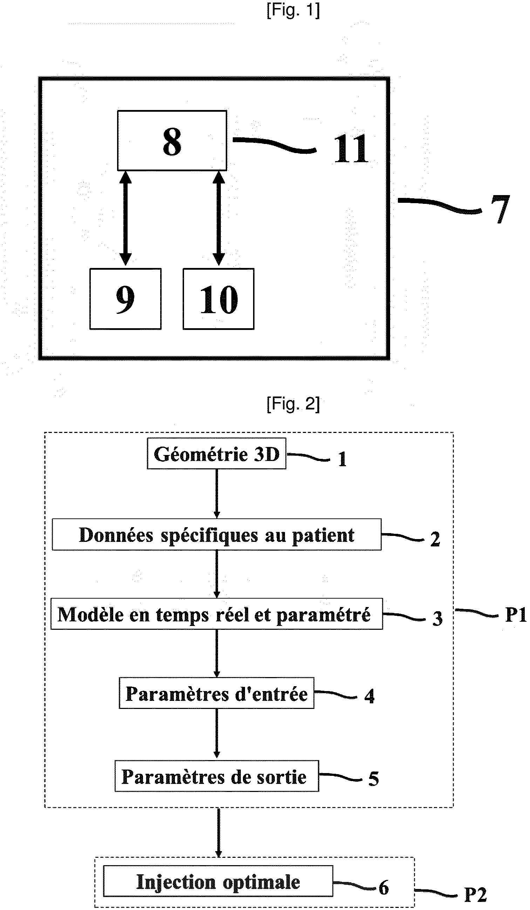

   [Fig. 1] est un synoptique des principaux blocs d'un dispositif programmable pour la mise en oeuvre du dispositif de l'invention ;
**Fig. 2**
   [Fig. 2] est un organigramme d'un procédé d'aide au ciblage de tumeur utilisant un dispositif selon l'invention ;
**Fig. 3**

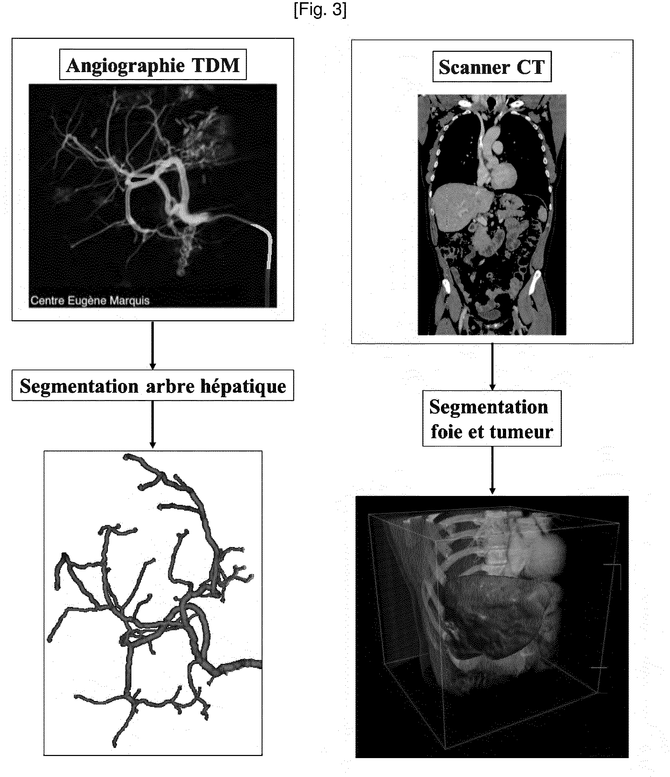

   [Fig. 3] est un exemple d'images des structures de l'arbre hépatique, du foie et de la tumeur obtenues par angiographie TDM ou scanner CT.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Il est maintenant fait référence à la figure 1.

La figure 1 illustre les principaux blocs d'un dispositif programmable 7 pour la mise en oeuvre du dispositif de l'invention. Il comprend une unité centrale de traitement 8, ou CPU, pour l'exécution des instructions, un écran 9, une unité 10 de saisie des commandes par opérateur, par exemple un clavier, une souris etc. Le dispositif 7 comprend éventuellement un contrôleur 11, permettant le contrôle à distance pour le dépannage ou le support immédiat de l'utilisateur

La figure 2 illustre les principales étapes mises en oeuvre dans un procédé d'aide au ciblage de la tumeur dans l'arbre hépatique selon un mode de réalisation de l'invention, mise en oeuvre par un processeur 8 d'un dispositif programmable 7. Le procédé comporte deux phases principales, une pour la planification P1, qui est effectuée pour obtenir toutes les données du patient et une phase P2 pour la modélisation du traitement. Comme illustré, la phase P1 consiste à son tour en cinq étapes. Une première étape 1 consiste à acquérir les images 3D spécifiques au patient et au cathéter. Par exemple, l'image 3D acquise contient l'arbre hépatique et la tumeur. Il peut s'agir d'une image 3D obtenue par Computed Tomography (CT). En variante, d'autres techniques comme l'IRM peuvent être utilisées.

L'arbre vasculaire est alors segmenté : un modèle anatomique tridimensionnel virtuel du patient est généré. Ce modèle est obtenu en appliquant des méthodes de traitement d'images à l'image 3D, via l'utilisation d'algorithmes automatiques ou semi-automatiques. Par exemple, l'angiographie TDM à faisceau conique 3D peut être acquise pendant la phase artérielle et l'extraction des vaisseaux à partir des images peut être obtenue par un pré-traitement consistant à rehausser les régions vasculaires, comme par exemple un filtre multi-échelle basé sur la matrice Hessienne comme le filtre 3D de Frangi, suivi d'un seuillage et d'une analyse de connectivité.. Ensuite l'estimation précise du volume de l'organe et de l'emplacement et du volume de la tumeur est obtenue à partir des images obtenues du scanner CT. Par exemple, la forme de l'organe peut être extraite avec une méthode de surface active semiautomatique intégrée qui effectue une segmentation 3D rapide du volume.

Enfin, les données spécifiques au patient sont acquises (étape 2). Ainsi, la pression dans des branches convenablement choisies de l'arbre vasculaire peut être mesurée avec un capteur de pression ; et la vitesse à l'entrée de l'arbre peut être mesurée par IRM.

L'étape 3 concerne la création d'un maillage de points à partir du modèle anatomique créé, afin de pouvoir effectuer des simulations de flux. La géométrie obtenue est paramétrée en fonction de la position et de la géométrie du cathéter, et des conditions aux limites. Dans le cadre de cet exemple de réalisation, la méthode des volumes finis est utilisée pour la simulation et le maillage est construit automatiquement avec des éléments tétraédriques.

En variante, il est possible d'obtenir un maillage structuré constitué d'éléments polygonaux et d'une couche limite définie de manière appropriée.

Un modèle de fluide dynamique et de transport de masse spécifique du patient est alors généré en définissant les propriétés rhéologiques du sang, les conditions aux limites spécifiques du patient, les paramètres de l'injection et le réglage nécessaire pour effectuer une simulation robuste et rapide.

Les conditions aux limites spécifiques du patient peuvent par exemple comprendre des valeurs de pression artérielle ou des valeurs de débit sanguin à chaque sortie de l'arbre vasculaire.

L'étape de définition de paramètres d'entrée (4) concerne le choix et la définition des paramètres connus qui constitueront des invariants. Dans le cas de cet exemple de réalisation, il s'agit de la géométrie du cathéter, tels que le diamètre et la longueur, du type de cathéter et de son positionnement, de la vitesse d'injection, et du type d'agent injecté.

L'étape de définition des paramètres de sortie (5) concerne le choix des paramètres cibles à optimiser. Dans le cas de cet exemple de réalisation, il s'agit de la quantité d'agent libérée dans la zone cible. Un nombre approprié de simulations sont donc automatiquement effectuées grâce à l'utilisation des modèles d'ordre réduit : l'effet de chaque paramètre du modèle (par exemple, diamètre du cathéter, vitesse d'injection, etc.) ou une combinaison de ceux-ci sur la distribution de l'agent dans la tumeur et le tissu sain peut être évalué. Des surfaces de réponse peuvent être fournies à l'utilisateur afin de comprendre qualitativement et immédiatement l'influence de chaque paramètre.

La phase P2 consiste à obtenir en temps réel les configurations d'injection optimales par la simulation spécifique au patient (6). À partir de la géométrie et du modèle paramétré, cette étape peut prévoir soit l'utilisation des calculs off-line effectués par les modèles d'ordre réduit soit l'utilisation de méthodes de type Lattice-Boltzmann. De cette façon, les configurations d'injection optimales seront fournies au clinicien.

La figure 3 illustre un exemple d'images pouvant être obtenues.

### Application industrielle

L'invention ne se limite pas aux exemples liés au traitement de CHC décrits ci-avant, seulement à titre d'exemple, mais elle englobe toutes les variantes que pourra envisager l'homme de l'art dans le cadre de la protection recherchée. En particulier, tous les traitements intraartériels pourront bénéficier avantageusement de l'invention sans qu'il soit nécessaire d'effectuer des adaptations trop importantes.

## Revendications

1. Dispositif médical comprenant une unité de commande configurée pour déterminer et afficher dynamiquement sur un dispositif d'affichage une information relative à une injection d'un agent actif dans un cathéter positionné ou positionnable dans un patient, l'information étant déterminée en fonction de :
- au moins une modélisation géométrique d'un arbre vasculaire du patient, alimentant une ou plusieurs tumeurs,
- au moins un paramètre interventionnel susceptible d'évoluer de façon dynamique et choisi parmi un type de cathéter, une position de cathéter, une vitesse d'injection, un type d'agent actif, une quantité d'agent actif et le nombre d'injections.

2. Dispositif médical selon la revendication 1 dans lequel l'agent actif est choisi parmi des particules comportant de l'Yttrium 90, des particules comportant de l'Holmium 166, et un fluide comportant du Rhenium 188.

3. Dispositif médical selon la revendication 1 ou 2 dans lequel le cathéter est choisi parmi un cathéter de type smart micro-cathéter, et un cathéter anti-reflux.

4. Dispositif médical selon l'une quelconque des revendications 1 à 3 dans lequel l'information est également déterminée en fonction de données mesurées sur le patient et de préférence choisies parmi une pression artérielle, une vélocité du sang et une distribution d'un agent actif.

5. Dispositif médical selon l'une quelconque des revendications 1 à 4, configuré pour déterminer l'information lorsque le cathéter n'est pas positionné dans le patient, de préférence dans lequel l'information est une position du cathéter.

6. Dispositif médical selon l'une quelconque des revendications 1 à 5, configuré pour déterminer l'information lorsque le cathéter est positionné dans le patient, de préférence dans lequel l'information est une quantité d'agent actif libéré.

7. Dispositif médical selon l'une quelconque des revendications 1 à 6, dans lequel le patient est porteur d'une ou plusieurs tumeurs, de préférence un carcinome hépatocellulaire.

8. Dispositif médical selon l'une quelconque des revendications 1 à 7, dans lequel l'unité de commande est configurée pour mettre en oeuvre un modèle de mécanique des fluides et de transport de masse.

9. Dispositif médical selon l'une quelconque des revendications 1 à 8, dans lequel l'information est déterminée en prenant en compte un résultat d'au moins une simulation réalisée par une méthode d'analyse de préférence par des volumes finis ou par des éléments finis ou par des modèles Lattice-Boltzmann.

10. Dispositif médical selon l'une quelconque des revendications 1 à 9, dans lequel l'information est déterminée en prenant en compte un modèle d'ordre réduit.

11. Dispositif médical selon l'une quelconque des revendications 1 à 10 dans lequel l'information est également déterminée en fonction d'images du patient, les images étant de préférence obtenues par CT ou par IRM ou par Imagerie Nucléaire ou par ultrasons.

12. Dispositif médical selon l'une quelconque des revendications 1 à 11, dans lequel l'information déterminée est choisie parmi des combinaisons des paramètres d'injection qui permettent le ciblage le plus efficace de la tumeur.

13. Programme informatique comportant des instructions pour la détermination et l'affichage dynamique sur un dispositif d'affichage d'une information relative à une injection d'un agent actif dans un cathéter positionné ou positionnable dans un patient, l'information étant déterminée en fonction de :
- au moins une modélisation géométrique d'un arbre hépatique du patient,
- au moins un paramètre chirurgical susceptible d'évoluer de façon dynamique et choisi parmi un type de cathéter, une position de cathéter, une vitesse d'injection, un type d'agent actif et une quantité d'agent actif ;
lorsque ce programme est exécuté par au moins un processeur.

14. Support d'enregistrement non transitoire lisible par un ordinateur sur lequel est enregistré un programme selon la revendication 13.

15. Procédé pré-opératoire d'aide à la décision médicale comportant une étape de détermination d'une information relative à une injection d'un agent actif dans un cathéter positionnable dans un patient, l'information étant déterminée en fonction de :
- au moins une modélisation géométrique d'un arbre hépatique du patient,
- au moins un paramètre chirurgical susceptible d'évoluer de façon dynamique et choisi parmi un type de cathéter, une position de cathéter, une vitesse d'injection, un type d'agent actif et une quantité d'agent actif.
